# EUROPÄISCHE PATENTSCHRIFT

(11) **EP 1 906 920 B1**
(45) Veröffentlichungstag und Bekanntmachung des Hinweises auf die Patenterteilung: **30.03.2011**
(21) Anmeldenummer: 06766055.5
(22) Anmeldetag: 08.07.2006
(51) Int. Cl.: A61K 9/00

(54) **PHARMAZEUTISCHE ZUSAMMENSETZUNG ZUR BEHANDLUNG VON XEROSTOMIE**
PHARMACEUTICAL COMPOSITION FOR TREATING XEROSTOMIA
COMPOSITION PHARMACEUTIQUE POUR TRAITER UNE XEROSTOMIE

(30) Priorität: 15.07.2005 CH 11782005
(43) Veröffentlichungstag der Anmeldung: 09.04.2008
(73) Patentinhaber: Bardazzi, Stefano, 1006 Lausanne (CH)
(72) Erfinder: Bardazzi, Stefano, 1006 Lausanne (CH)
(74) Vertreter: Grosfillier, Philippe
(86) Internationale Anmeldenummer: PCT/IB2006/052317
(87) Internationale Veröffentlichungsnummer: WO 2007/010430

(56) Entgegenhaltungen:
- EP-A2- 0 875 244
- WO-A2-01/13956
- WO-A2-96/34596
- US-B1- 6 949 262

## Beschreibung

### Technisches Gebiet

Die vorliegende Erfindung bezieht sich auf das Gebiet der Behandlung von Mundtrockenheit ( Xerostomie ), wie sie insbesondere infolge des Sjögren-Syndroms auftritt.

Beim Sjögren-Syndrom handelt es sich um eine Krankheit, welche eine chronische Entzündung in den Tränen-und Speicheldrüsen verursacht. Die Produktion von Tränenflüssigkeit und Speichel ist dabei stark reduziert oder kommt sogar ganz zum Erliegen, was trockene Augen sowie einen trockenenen Mund verursacht. Das Hauptproblem is hierbei, dass die Patienten ständig trinken müssen, um ihren Mund-und Rachenraum feucht zu halten, um überhaupt in der Lage zu sein, zu sprechen oder zu essen.

Weitere Ursachen für Mundtrockenheit sind beispielsweise Bestrahlungstherapien, die Einnahme bestimmter Arzneimittel wie z.B. Anti-Depressiva, ggf. aber auch nur eine zu trockene Umgebungsluft.

Dehydrierte Schleimhäute werden leicht beschädigt und bilden dann die ideale Basis für eine starke mikrobiologische Vermehrung.

### Stand Der Technik

Es sind bereits verschiedene Mittel gegen Mundtrockenheit in Form von Mundsprays/Hydrogels bekannt und auf dem Markt erhältlich. Das Produkt EMOFLUOR® der DR. Wild & Co. AG, Basel dient zur Mundbefeuchtung und enthält nebenbei auch noch Fluor, dies allerdings nur als Schutz gegen Karies. Die Produkte ORALBALANCE® der Firma Laclede Inc., Rancho Doninguez, California, USA, sowie GLANDOSAN® der Firma Helvepharm AG, Frauenfeld dienen als künstlicher Speichelersatz. Keines dieser Mittel enthält eine aktive Substanz mit pharmazeutischer Wirkung.

Daneben st Emu-Oel, gewonnen als Naturprodukt von den in Australien beheimateten Emu-Laufvögel (Dromaius nova hollandiae), als ein Mittel bekannt insbesondere zur oberflächlichen Behandlung der Haut bei Verletzungen, HauterkrankunGen (WO 96134596) sowie aus kosmetischen Gründen (EP 0875244). Die WO92/08470 erwähnt Emu-Oel in Verbindung mit einem sogenannten Transportverstärker auch zur mukosalen Verabreichung, führt das aber nicht weiter aus. Generell werden dem- Emu-Oel unter anderem anti-septische, anti-entzündliche sowie anti-virale Eigenschaften zugeschrieben. Z.B. WO 01/13956 beschreibt die Verwendung von Emu-Oel zur Behandlung von Pilzinfektionen, backterielle oder virale.

Die Wirkung des Emu-Oels beruht vermutlich auf seiner speziellen Zusammensetzung. Es besteht fast aus 100% Tryglyceriden, ist frei von Phospholipiden und deshalb als vollständig neutrales Lipid angesehen werden. Es enthält einen hohen Anteil ( ca. 25 %) an ungesättigten essentiellen Fettsäuren wie Omega 3 und Omega 6, die der Körper für den Aufbau verschiedener wichtiger Stoffe wie z.B. von Hormonen benötigt und die bei Zellaktivitäten wie Entzündungs-, Heilungs- oder Wachstumprocessen beteiligt sind.

### Darstellung Der Erfindung

Gegenstand der Erfindung ist die Verwendung von Emu-Oel zur Herstellung einer Pharmazeutischen Zusammensetzung (Präparat) zur Behandlung von Mundtrockenheit, insbesondere von einer Mundtrockenheit infolge des Sjögren Syndorme und/oder einer Bestrahlungstherapie und/oder einer Einnahme von Medikamenten wie z.B. Anti-Depressiva.

Die erfindungsgemäss auf der Basis von Emu-Oel hergestellete Pharmazeutische Zusammensetzung (Präparat) setzt sich an der Schleimhäuten im Mund-und Rachenraum fest. Wegen des daraufhin durch das Emu-Oel verursachten, wesentlich reduzierten trans-epidermalen Wasserverlustes ( Trans-Epiderma-Water-Loss (TEWL)) wird das Austrocknen des Mund-und Rachenraumes erheblich reduziert, so dass das Mittel als öl-basierter

Feuchtigkeitsspender wirkt. Zusätzlich hat das Emu-Oel eine signifikante aktive antientzündende Wirkung sowie anti-virale Eigenschaften. Es wirkt auch abschwellend. Gegenüber anderen bekannten Präparate weist die erfindungsgemässe Pharmazeutische Zusammensetzung auch eine wesentlich verlängerte Wirkungsdauer auf.

### BEISPIEL

Nachfolgend wird ein Formulierungsbeispiel für eine Pharmazeutische Zusammensetzung (Präparat) in Form eines Hydrogels nach der Erfindung angegeben. Zunächst werden unabhängig voneinander drei Phasen zubereitet:

### Emu-Oel Phase I:

| | | |
|---|---|---|
| - | Emu-Oel | 100 ml |
| - | BHA ( butylated hidrocyanisol) | 0.02 g |
| - | Benzoesäure | 0.1 g |
| - | Saccharin | 0.1 g |
| - | Minz-Oel | 5 Tropfen |

Die vorgenannten Zutaten werden gut miteinander vermischt.

### Delious Phase II:

| | | |
|---|---|---|
| - | Delios VI | 100 ml |
| - | BHA (butylated hidrocyoanisol) | 0.02 g |
| - | Benzoesäure | 0.1 g |
| - | Saccharin | 0.1 g |
| - | Minz-Oel | 5 Tropfen |

Die vorgenannten Zutaten werden gut miteinander vermischt.

### Wässrige Phase:

| | | |
|---|---|---|
| 1. | Wasser | 100 ml |
| 2. | Methylzellulose | 4.5 g |
| 3. | Benzoesäure | 0.1 g |
| 4. | EDTA | 0.01 g |
| 5. | Ascorbinsäure | 0.1g |
| 6. | Saccharin | 0.1g |
| 7. | Minz-Oel | 5 Tropfen |

Die Zutaten 2. - 7. werden dem Wasser 1. beigegeben. Danach lässt man die Zellulose 24 Stunden quellen.

Nach Ablauf der Quellzeit werden 120 ml der Emu-Oel Phase I mit 40 ml der Delios Phase I und 240 ml der wässrigen Phase gut vermischt.

Die resultierende Flüssigkeit wird in einen Behälter von z.B. 30 ml abgefullt, welcher mit einer aufgeschraubten Pumpspraykappe versehen ist. Die Pumpspraykappe ist so konzipiert, dass pro Sprayhub eine Flüssigkeitsmenge von ca. 0.2 ml - 0.3 ml in Form eines Aerosols abgegeben wird und zwar vorzugsweise unter einem raltiv weiten Spühwinkel, der Mund- und Rachneraum grossflächig zu besprühen gestattet.

Es hat sich als vorteilhaft erwiesen, die Viskosität der Pharmazeutische Zusammensetzung in Form von Hydrogel, etwa auf einen Wert im Bereich 100 mPa s-1000 mPa s einzustellen, damit es nach Applikation ausreichend lange an der Schleimhaut festhält.

Es hat sich auch als besonders vorteilhaft erwiesen, als Trägersubstanz LUTROL F127^{™} und/oder LUTROL F68^{™} (also genannt POLOXAMER 407 und/oder POLOXAMER 188), allein oder zusammen mit CARBOPOL™ zu benutzen.

Diese Trägersubstanz kann übrigens auch mit anderen Wirkstoffen benützt werden, die mündlich anwendbar sind.

Verschiedene Emu-Öl Rezepturen (OralEmu) wurden vom Institut für Biopharmazie und Galenik der Universität Camerino (I) entwickelt und von der Abteilung für medizinische und chirurgische Wissenschaft der Universität Padova (I), sowie dem regionalen Gesundheitsversorger ULSS ebenfalls in Padova (I), getestet.

Die neuste klinische Studie durchgeführt vom ULSS-Padova an 56 Patienten mit schwerer Mundtrockenheit in Folge des Sjögren Syndrom, untermauert die Wirksamkeit des OralEmu-Mundsprays.

Mehr als 70% der Patienten bestätigten eine leichte bis sehr gute Besserung. Mundtrockenheit, Brennen und Entzündung des Mund und Rachenraumes verbesserten sich um 36% bis 43% bei mehr als der Hälfte der Patienten.

70% der an der Studien teilnehmenden Patienten würden das OralEmu-Präparat, Drittpersonen mit ähnlichen Symptomen weiterempfehlen. Die im Durchschnitt mehr als 90 Minuten andauernde Wirkung des OralEmu wurde für eine Mundsprayapplikation als sehr akzeptabel empfunden.

## Patentansprüche

1. Verwendung von Emu-Oel zur Herstellung einer Pharmazeutischen Zusammensetzung zur Behandlung von Mundtrockenheit.

2. Verwendung von Emu-Oel gemäss Anspruch 1 zur Herstellung einer Pharmazeutischen Zusammensetzung zur Behandlung von Mundtrockenheit infolge einer Bestrahlungstherapie.

3. Verwendung von Emu-Oel gemäss Anspruch 1 zur Herstellung einer Pharmazeutischen Zusammensetzung zur Behandlung von Mundtrockenheit infolge des Sjögren Symdroms.

4. Verwendung von Emu-Oel gemäss Anspruch 1 zur Herstellung einer Pharmazeutischen Zusammensetzung zur Behandlung von Mundtrockenheit infolge Einnahme von Medikamenten wie z.B Anti-Depressiva.

5. Verwendung von Emu-Oel nach einem der vorangegangenen Ansprüche zusammen mit, als Trägersubstanz, Poloxamer 407 und/oder Poloxamer 188, allein oder zusammen mit einem Carbomer.

6. Verwendung von poloxamer 407 und/oder poloxamer 188, allein oder zusammen mit einem Carbomer, als Trägersubstanz für eine mündliche Anwendung.

## Claims

1. Use of emu oil for producing a pharmaceutical composition for treating dry mouth.

2. Use of emu oil according to Claim 1 for producing a pharmaceutical composition for treating dry mouth resulting from radiotherapy.

3. Use of emu oil according to Claim 1 for producing a pharmaceutical composition for treating dry mouth resulting from Sjögren's syndrome.

4. Use of emu oil according to Claim 1 for producing a pharmaceutical composition for treating dry mouth resulting from intake of drugs such as, for example, anti-depressants.

5. Use of emu oil according to any of the preceding claims together with, as a carrier, poloxamer 407 and/or poloxamer 188, alone or together with a carbomer.

6. Use of poloxamer 407 and/or poloxamer 188, alone or together with a carbomer, as a carrier for oral use.

## Revendications

1. Utilisation d'huile d'émeu pour la fabrication d'une composition pharmaceutique destinée au traitement de la sécheresse de la bouche.

2. Utilisation d'huile d'émeu selon la revendication 1 pour la fabrication d'une composition pharmaceutique destinée au traitement de la sécheresse de la bouche faisant suite à une radiothérapie.

3. Utilisation d'huile d'émeu selon la revendication 1 pour la fabrication d'une composition pharmaceutique destinée au traitement de la sécheresse de la bouche faisant suite au syndrome de Sjögren.

4. Utilisation d'huile d'émeu selon la revendication 1 pour la fabrication d'une composition pharmaceutique destinée au traitement de la sécheresse de la bouche faisant suite à une prise de médicaments comme par exemple des antidépresseurs.

5. Utilisation d'huile d'émeu selon l'une quelconque des revendications précédentes, conjointement avec, comme substance véhicule, du poloxamère 407 et/ou du poloxamère 188, seuls ou conjointement avec un carbomère.

6. Utilisation de poloxamère 407 et/ou de poloxamère 188, seuls ou conjointement avec un carbomère, pour une application buccale.
